# EUROPÄISCHE PATENTSCHRIFT

(11) **EP 3 743 024 B1**
(45) Veröffentlichungstag und Bekanntmachung des Hinweises auf die Patenterteilung: **09.08.2023**
(21) Anmeldenummer: 19717429.5
(22) Anmeldetag: 06.03.2019
(51) Int. Cl.: A61F 2/14, A61F 9/007, G02C 7/04

(54) **OPHTHALMOLOGISCHES OPERATIONSSET MIT EINER KONTAKTLINSE**
OPHTHALMOLOGICAL SURGICAL SET INCLUDING A CONTACT LENS
KIT CHIRURGICAL OPHTALMOLOGIQUE AVEC LENTILLE DE CONTACT

(30) Priorität: 12.03.2018 DE 102018203695
(43) Veröffentlichungstag der Anmeldung: 02.12.2020
(73) Patentinhaber: Geuder AG, 69126 Heidelberg (DE)
(72) Erfinder: EL-AYARI, Hamadi, 60323 Frankfurt (DE)
(74) Vertreter: Ullrich & Naumann PartG mbB
(86) Internationale Anmeldenummer: PCT/DE2019/200021
(87) Internationale Veröffentlichungsnummer: WO 2019/174683

(56) Entgegenhaltungen:
- EP-A1- 1 561 440
- WO-A1-2017/077300
- GB-A- 2 333 456
- US-A1- 2007 142 828
- US-A1- 2014 039 377
- US-A1- 2015 209 181

## Beschreibung

Die Erfindung betrifft ein ophthalmologisches Operationsset. Des Weiteren betrifft die Erfindung eine Kontaktlinse zur Verwendung in einem solchen ophthalmologischen Operationsset.

Aus der Praxis sind unterschiedliche Methoden zur Korrektur einer Fehlsichtigkeit vorbekannt. Die einfachste Möglichkeit stellen hierbei Brillen und Kontaktlinsen dar. Diese werden von den Nutzern jedoch oftmals als äußerst störend bzw. unkomfortabel empfunden und sind in Anschaffung und Unterhalt teuer.

Im Rahmen der refraktiven Chirurgie wird die Gesamtbrechkraft des optischen Systems des Auges derart optimiert, dass die Umwelt scharf auf der Netzhaut abgebildet wird, so dass im Idealfall auf eine Sehhilfe verzichtet werden kann. Die Mehrzahl der refraktiven Eingriffe stellen Laserverfahren dar, beispielsweise die Laser-in-situ-Keratomileusis (LASIK) und die Laser-epitheliale Keratomileusis (LASEK). Bei diesen Verfahren wird Gewebe von der Hornhaut (Cornea) abgetragen, um die Hornhautkrümmung zu verändern.

Sämtliche Methoden der refraktiven Chirurgie weisen den Nachteil auf, dass es sich um einen invasiven Eingriff handelt. Dieser geht mit entsprechenden Risiken einher, insbesondere dem Auftreten von Entzündungen, einem Schwächen der Cornea oder der FLAP verwächst nicht mehr. Des Weiteren sind solche Eingriffe für den Patienten mit Schmerzen verbunden.

Weiterhin ist es aus der Praxis bekannt, Fehlsichtigkeit durch die sogenannte Orthokeratologie zu behandeln. Dabei wird für den Patienten eine speziell angepasste, formstabile Kontaktlinse hergestellt, die an im Vorfeld bestimmten Stellen Druck auf die Cornea ausübt. Durch diesen Druck wird die Cornea gezielt verformt, so dass sich die Brechung verändert. Entsprechende Kontaktlinsen werden lediglich meist nur über Nacht getragen, so dass der Anwender tagsüber ohne Sehhilfe auskommt. Solche Orthokeratologie-Kontaktlinsen sind beispielsweise aus den folgenden Dokumenten bekannt:
US2007/142828, US2015/209181, EP1561440 oder US2014/039377.

Ein wesentlicher Nachteil dieser Methode liegt darin, dass es sich um eine zeitlich eingeschränkte Korrektion der Fehlsichtigkeit handelt, d.h. der Vorgang der Corneaverformung ist reversibel. Über den Tag hinweg verschlechtert sich die Sehkraft wieder, so dass beispielsweise Autofahren am späten Abend ohne Sehhilfe zu vermeiden ist. Auch müssen die Kontaktlinsen im Regelfall jede Nacht getragen werden.

Der vorliegenden Erfindung liegt daher die Aufgabe zugrunde, mit konstruktiv einfachen Mitteln und für den Patienten schonend eine Korrektur von Fehlsichtigkeit zu ermöglichen.

Erfindungsgemäß wird die voranstehende Aufgabe durch die Merkmale des Anspruches 1 gelöst. Danach umfasst ein ophthalmologisches Operationsset mindestens eine Kontaktlinse und mindestens ein Vernetzungsmittel für die Cornea, wobei eine der Cornea zugewandte Verformungsseite der Kontaktlinse als Negativ einer zumindest im Wesentlichen emmetropen Corneaform ausgebildet ist und wobei die Kontaktlinse Mittel zur Bereitstellung von Unterdruck zwischen der Kontaktlinse und dem die Kontaktlinse tragenden Auge aufweist, so dass die Cornea an die Verformungsseite ansaugbar und somit in die zumindest im Wesentlichen emmetrope Corneaform bringbar ist. Das Vernetzungsmittel liegt weiterhin als Teil einer pharmazeutischen Zusammensetzung vor, die für die Verabreichung an bzw. auf das Auge geeignet ist, und folgende Komponenten umfasst:
(i) einen wässrigen Träger; und (ii) das nicht-toxische, wasserlösliche Vernetzungsmittel, umfassend zwei oder mehrere Carboxylgruppen und/oder eine oder mehrere Anhydridgruppen und/oder eine oder mehrere Estergruppen oder ein pharmazeutisch annehmbares Salz davon, gelöst in dem wässrigen Träger; wobei die Zusammensetzung einen pH im Bereich von 6 bis 9 aufweist, wobei das nicht-toxische, wasserlösliche Vernetzungsmittel nicht Glutarsäureanhydrid ist.

In erfindungsgemäßer Weise ist zunächst erkannt worden, dass eine Korrektur der Fehlsichtigkeit in verblüfFend einfacher Weise erfolgen kann, indem die Cornea in eine gewünschte, emmetrope Form gebracht wird und in dieser mittels eines Vernetzungsmittels gehalten wird. Dazu ist in weiter erfindungsgemäßer Weise eine Kontaktlinse vorgesehen, die im Wesentlichen wie eine orthokeratologische Kontaktlinse wirkt, wobei Mittel vorgesehen sind, um zwischen der Verformungsseite der Kontaktlinse und der Cornea einen Unterdruck zu erzeugen. Dadurch ist es möglich, die Cornea an die Verformungsseite anzusaugen und somit in kürzester Zeit die gewünschte, zumindest im Wesentlichen emmetrope Corneaform zu erzielen. Um die Cornea in diesem Zustand zu halten bzw. "einzufrieren", wird im Vorfeld das Vernetzungsmittel auf die Cornea aufgebracht werden. Das Vernetzungsmittel muss dabei nicht in Reinform vorliegen, kann es sich vielmehr um eine pharmazeutische Zusammensetzung handeln, die neben mindestens einem Vernetzungsmittel auch ein oder mehrere andere Komponenten enthält. Während die Cornea durch den Unterdruck in der gewünschten Form gehalten ist, erfolgt der durch das Vernetzungsmittel hervorgerufene "Cross-Linking-Vorgang", das heißt, die Cornea vernetzt, wodurch sich der biomechanische Widerstand (ähnlich dem Elastizitätsmodul) der Cornea drastisch zu erhöht. Die Mittel können als Anschluss für ein Fluidverbindungselement ausgebildet sein, so dass über das Fluidverbindungselement, beispielsweise einen Schlauch, Unterdruck zwischen der Verformungsseite und der Cornea realisierbar ist.

Durch das erfindungsgemäße Operationsset ist es somit möglich, die Cornea in kurzer Zeit in einen Soll-Zustand zu bringen und in dieser emmetropen Form zu halten bzw. zu fixieren, ohne dass die Kontaktlinse über einen längeren Zeitraum oder in regelmäßigen Abständen zu tragen ist.

Unter der emmetropen Corneaform ist dabei die auf den Patienten individuell angepasste Form der Cornea zu verstehen, bei der Normalsichtigkeit besteht bzw. bei der korrigierbare Fehlsichtigkeiten zumindest im Wesentlichen behoben sind. Der Begriff "Normalsichtigkeit" ist dabei im weitesten Sinne zu verstehen und nicht zwangsweise mit einer 100 prozentigen Sehschärfe gleichzusetzen. Des Weiteren wird darauf hingewiesen, dass es sich bei dem Auge insbesondere um ein menschliches Auge handeln kann. Weiterhin ist der Begriff "Operation" im weitesten Sinne zu verstehen, kann es sich dabei um ein einen Eingriff durch einen Arzt oder auch einem Optiker handeln, durch den eine Fehlsichtigkeit behoben bzw. verbessert werden soll.

Im Hinblick auf eine Erzeugung des Unterdrucks mit einfachen Mitteln kann das Operationsset eine Spritze mit einem Fluidverbindungselement, insbesondere einer Schlauchverbindung umfassen, wobei der Anschluss der Kontaktlinse mit der Fluidverbindungselement strömungsverbindungbar ist. Generell kann jede Form einer Zylinder-Kolben-Einrichtung oder einer beliebigen anderen Einrichtung zur Bereitstellung von Unterdruck über eine Schlauchverbindung mit dem Anschluss verbindbar sein.

In vorteilhafter Weise kann die Spritze, insbesondere der Kolben der Spritze, über ein elastisches Element vorgespannt sein, so dass ein definierter Unterdruck erzeugbar ist. Bei dem elastischen Element kann es sich beispielsweise um eine Feder, insbesondere eine Druckfeder, handeln. Beispielsweise kann der Spitzenkolben derart vorgespannt sein, dass dieser aus dem Spritzenzylinder herausgedrückt wird. Über das Volumen des Spritzenzylinders lässt sich der Unterdruck vorbestimmen, wozu auf die thermische Zustandsgleichung idealer Gase p x V = n × R × T verwiesen wird.

Zur Realisierung einer Verbindung zwischen dem Anschluss und einem Fluidverbindungselement, insbesondere einem Schlauch, kann der Anschluss der Kontaktlinse und/oder das Fluidverbindungselement derart ausgebildet sein, dass eine form- und/oder kraftschlüssige Verbindung herstellbar ist. Beispielsweise kann der Anschluss als Teil einer Bajonettverbindung oder eines Luer-Anschlusses realisiert sein. Das Fluidverbindungselement kann ein damit korrespondierendes Verbindungselement aufweisen.

Im Hinblick auf eine exakte Positionierung bzw. Ausrichtung der Kontaktlinse in dem Auge des Patienten kann an der Kontaktlinse mindestens eine Markierung vorgesehen sein. Die Markierung kann beispielsweise in der Art eines Fadenkreuzes bzw. Absehens eines Zielfernrohrs realisiert sein. Zusätzlich ist die Nutzung weiterer Hilfsmittel, beispielsweise von Einspiegelungen der Soll-Position über ein OP-Mikroskop oder die Verwendung einer Spaltlampe denkbar.

In besonders vorteilhafter Weise kann die Verformungsseite der Kontaktlinse dazu ausgebildet sein, die Cornea derart zu verformen, dass mindestens eine Zone zur Korrektur von Fernsicht und mindestens eine Zone zur Korrektur von Nahsicht erzeugbar ist.

Das Vernetzungsmittel liegt als Teil einer pharmazeutischen Zusammensetzung vor, die für die Verabreichung an das Auge geeignet ist. Die Zusammensetzung umfasst gemäß der WO 2017/077300 A1:
(i) einen wässrigen Träger; und
(ii) das nicht-toxisches, wasserlösliches Vernetzungsmittel, umfassend zwei oder mehrere Carboxylgruppen und/oder eine oder mehrere Anhydridgruppen und/oder eine oder mehrere Estergruppen oder ein pharmazeutisch annehmbares Salz davon, gelöst in dem wässrigen Träger;
wobei die Zusammensetzung einen pH im Bereich von 6 bis 9 aufweisen kann; mit der Maßgabe, dass das nicht-toxische, wasserlösliche Vernetzungsmittel nicht Glutarsäureanhydrid sein kann.

In vorteilhafter Weise kann das nicht-toxische, wasserlösliche Vernetzungsmittel der pharmazeutischen Zusammensetzung zwei bis vier Carboxylgruppen umfassen.

In weiter vorteilhafter Weise kann das nicht-toxische, wasserlösliche Vernetzungsmittel eine Verbindung der nachstehend gezeigten Formel (I) oder Formel (II) oder ein pharmazeutisch annehmbares Salz und/oder Solvat davon sein 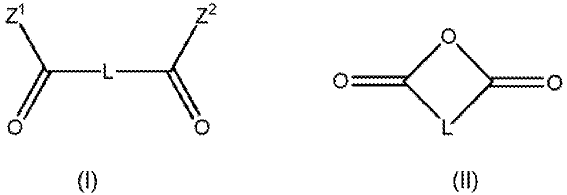 wobei:
L ausgewählt sein kann aus:
   (i) linearem oder verzweigtem (1-12 C)Alkylen-Linker, der gegebenenfalls ein oder mehrere Heteroatome, ausgewählt aus N, O oder S, umfasst und gegebenenfalls mit einer oder mehreren Gruppen substituiert ist, ausgewählten aus Carboxy, Anhydrid, Oxo, Halogen, Trifluormethyl, Cyano, Nitro, Hydroxy, Mercapto, Amino, (1-6 C)Alkyl, (1-6 C)Alkoxy, (1-6 C)Alkylthio, (1-6 C)Alkylsulfinyl, (1-6 C)Alkylsulfonyl, (1-6 C)Alkylamino, di-[(1-6 C)alkyl]-amino, (1-6 C)Alkoxycarbonyl, (2-6 C)Alkanoyl oder (2-6 C)Alkanoyloxy, oder
   (ii)einer wasserlöslichen Polymerkette; und
Z¹ und Z² unabhängig ausgewählt sein können aus OH oder OR, wobei R ausgewählt ein kann aus (1-6C)Alkyl, Succinimid, 3-Sulfosuccinimid oder Pentafluorphenyl.

In weiter vorteilhafter Weise kann das nicht-toxische, wasserlösliche Vernetzungsmittel eine Verbindung der Formel (I) oder Formel (II) oder ein pharmazeutisch annehmbares Salz und/oder Solvat davon sein: 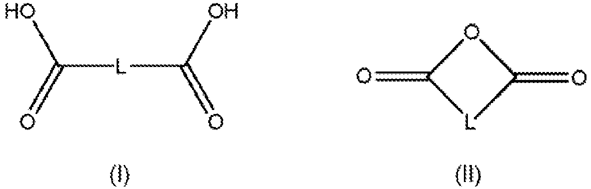 wobei L
(i) ein linearer oder verzweigter (1-12 C)Alkylen-Linker, der gegebenenfalls ein oder mehrere Heteroatome, ausgewählt aus N, O oder S, umfassen kann und gegebenenfalls mit einem oder mehreren Gruppen substituiert sein kann, ausgewählten aus Carboxy, Anhydrid, Oxo, Halogen, Trifluormethyl, Cyano, Nitro, Hydroxy, Mercapto, Amino, (1-6 C)Alkyl, (1-6 C)Alkoxy, (1-6 C)Alkylthio, (1-6 C)Alkylsulfinyl, (1-6 C)Alkylsulfonyl, (1-6 C)Alkylamino, di-[(1-6 C)alkyl]-amino, (1-6 C)Alkoxycarbonyl, (2-6 C)Alkanoyl oder (2-6 C)Alkanoyloxy, oder
(ii) eine wasserlösliche Polymerkette sein kann.

Gemäß einer weiteren vorteilhaften Ausgestaltung ist es denkbar, dass das nicht-toxische, wasserlösliche Vernetzungsmittel eine Verbindung der Formel (I) oder ein pharmazeutisch annehmbares Salz und/oder Solvat davon sein kann, und wobei L ein (2-8 C)Alkyl sein kann, das gegebenenfalls mit einer oder mehreren Gruppen der Formel -(CH₂)ₙCO₂H substituiert ist, wobei n eine ganze Zahl zwischen 0 und 10 sein kann.

Weiterhin kann die pharmazeutische Zusammensetzung derart realisiert sein, dass das nicht-toxische, wasserlösliche Vernetzungsmittel ausgewählt sein kann aus Sebacinsäure, Azelainsäure, Korksäure, Pimelinsäure, Adipinsäure, Glutarsäure oder Bernsteinsäure.

In vorteilhafter Weise kann das nicht-toxische, wasserlösliche Vernetzungsmittel der pharmazeutischen Zusammensetzung eine Verbindung der Formel (I) oder ein pharmazeutisch annehmbares Salz und/oder Solvat davon sein, und kann L eine Polyethylenglycolkette sein.

Bei der pharmazeutischen Zusammensetzung kann zusätzlich das nicht-toxische, wasserlösliche Vernetzungsmittel Bis-(Succinimidyl)-penta-(Ethylenglycol) sein.

Im Hinblick auf eine vorteilhafte Ausgestaltung ist es möglich, dass die pharmazeutische Zusammensetzung weiterhin einen Puffer umfasst, um den pH der Zusammensetzung in einem Bereich von pH 6 bis 9 zu halten. Im Konkreten kann der Puffer die Zusammensetzung in einem Bereich von pH 6,5 bis 7,5 halten. Alternativ oder zusätzlich kann die pharmazeutische Zusammensetzung weiterhin ein oder mehrere Peptidkopplungsreagenzien umfassen und/oder ein oder mehrere Carbodiimid-Peptidkopplungsreagenzien umfassen. Des Weiteren kann es von Vorteil sein, wenn die pharmazeutische Zusammensetzung ein Aktivierungsmittel umfasst.

Die nicht-toxischen, wasserlöslichen Vernetzungsmittel der vorliegenden Erfindung sind entweder vollständig oder teilweise

in einem wässrigen Träger gelöst. Unter einem "wässriger Träger" kann ein flüssiger Träger verstanden werden, der überwiegend Wasser enthält, beispielsweise mehr als etwa 50 Vol.-% Wasser.

Weiterhin ist es möglich, dass der wässrige Träger weitere Lösungsmittel enthält, beispielsweise organische Lösungsmittel, die vollständig oder teilweise mit Wasser mischbar sind. Der wässrige Träger kann auch Additive umfassen, die ionisch, organisch oder amphiphil sein können, insbesondere Tenside, Viskositätsmodifikatoren, Tonizitätsmittel, Sterilisationsmittel und Löslichkeitsverstärker.

Als Puffer kann ein beliebiger, geeignerter Puffer ausgewählt werden, beispielsweise aus der Gruppe umfassend: Phosphat-, Acetat-, Citrat-, Sulfonsäure-, Ascorbat-, Linolenat-, Carbonat- und Bicarbonat-basierte Puffer. Die Vernetzung von biologischem Material (z. B. Kollagen) durch die voranstehend beschriebenen pharmazeutischen Zusammensetzungen wird durch die Reaktion von Carboxyl- (oder Anhydrid-) Gruppen der Vernetzungsmittel mit Aminofunktionalitäten auf dem biologischen Material vermittelt. Dabei werden zwei oder mehrere Amidbindungen ausgebildet, so dass das biologische Material kovalent vernetzt wird.

Details konkreter Ausführungsbeispiele der pharmazeutischen Zusammensetzung können der zuvor genannten WO 2017/077300 A1 entnommen werden.

Zur Vereinfachung der Handhabung kann das Vernetzungsmittel bzw. die pharmazeutische Zusammensetzung als Vernetzungsflüssigkeit in einer Dosiereinrichtung in dem Operationsset angeordnet sein. Bei der Dosiereinrichtung kann es sich vorteilhafter Weise um eine Spritze, eine Pipette oder eine andere Vorrichtung handeln, die eine tröpfchenweise Applikation der Vernetzungsflüssigkeit ermöglicht.

Zur Verabreichung der pharmazeutischen Zusammensetzung kann die Dosiereinrichtung eine erste Kammer mit der pharmazeutischen Zusammensetzung aufweisen und eine zweite Kammer mit ein oder mehreren Peptidkupplungsreagenzien und gegebenenfalls einen Aktivator, gelöst in einem geeigneten pharmazeutisch annehmbaren Träger aufweisen, wobei die Dosiereinrichtung so konfiguriert ist, dass sie zumindest einen Teil des Inhalts der ersten und zweiten Kammer entweder vor oder während der Abgabe an das Auge mischt.

In weiter vorteilhafter Weise kann das Operationsset mindestens einen Lidsperrer und/oder mindestens ein Aufnahmemittel - beispielsweise einen Tupfer - für überschüssige Flüssigkeiten bzw. überschüssiges Vernetzungsmittel von dem Auge abzutragen.

Eine Kontaktlinse zur Verwendung in einem ophthalmologischen Operationsset nach einem der Ansprüche 1 bis 13 könnte mit einer der Cornea zugewandte Verformungsseite, die als Negativ einer zumindest im Wesentlichen emmetropen Corneaform ausgebildet ist und mit Mitteln zur Bereitstellung von Unterdruck zwischen der Verformungsseite und dem die Kontaktlinse tragenden Auge angegeben werden, so dass die Cornea an die Verformungsseite ansaugbar und somit in die zumindest im Wesentlichen emmetrope Corneaform bringbar ist.

Die Kontaktlinse kann im Spritzgussverfahren bzw. Mikrospritzgussverfahren oder auch durch spanende Verfahren, beispielsweise Drehen oder Fräsen, oder auch mittels 3D-Druckverfahren hergestellt werden.

Es wird weiterhin ein Verfahren zur Behandlung von Fehlsichtigkeit, insbesondere unter Verwendung eines ophthalmologischen Operationssets nach einem der Ansprüche 1 bis 13 beschrieben, welches nicht Teil der Erfindung ist. Dieses Verfahren umfasst die folgenden Schritte:
- Aufbringen eines Vernetzungsmittels auf das zu behandelnde Auge,
- Einsetzen der Kontaktlinse auf das zu behandelnde Auge und Erzeugung eines Unterdrucks, so dass die Cornea an die Verformungsseite der Kontaktlinse angesaugt wird,
- Halten des Unterdrucks, bis die Vernetzung zumindest im Wesentlichen abgeschlossen ist,
- Entfernen der Kontaktlinse von dem zu behandelnden Auge.

Weiterhin ist es denkbar, dass dem Patienten zunächst eine Lidsperre eingesetzt wird und ggf. die Cornea trocken getupft wird, um das aufzubringende Vernetzungsmittel nicht zu verdünnen. Auch ist es möglich, dass der Eingriff unter Anästhesie erfolgt, sollte der Patient das wünschen oder das Auge nicht ruhig halten können.

Nachdem die Kontaktlinse aus dem Auge entfernt ist, kann es notwendig sein, etwaiges verbliebenes Vernetzungsmittel bzw. Reagenzien auszuspülen.

Sollte der Patient seine Fehlsichtigkeit bisher durch Kontaktlinsen ausgleichen, kann es von Vorteil sein, bei weichen Kontaktlinsen diese mindestens 1 Woche vor einer Voruntersuchung nicht einzusetzen, bei harten Kontaktlinsen mindestens 2 Wochen davor, um das Untersuchungsergebnis nicht zu verfälschen. Die Voruntersuchung kann insbesondere eine Tonometrie umfassen, bei der eine Messung des Augeninnendrucks zum Glaukomausschluss (Grüner Star) erfolgt. Alternativ oder zusätzlich kann eine Netzhautdiagnostik durchgeführt werden, um eventuell vorhandene Degenerationen der Netzhaut sowie Schwachstellen, insbesondere am Netzhautrand, festzustellen. Dazu können die Pupillen mittels Augentropfen erweitert werden. Alternativ oder zusätzlich können Visus und Refraktion bestimmt werden, d.h. eine objektive und subjektive Bestimmung der Brechkraft der zu behandelnden Augen (Dioptrienzahl). Alternativ oder zusätzlich kann eine Pachymetrie, d.h. eine Messung der Hornhautdicke per Ultraschalluntersuchung erfolgen und/oder eine Biometrie zur Vermessung der Länge des Augapfels per Lasertechnik (IOL Master) durchgeführt werden. Gemäß einer weiteren alternativen oder zusätzlichen Ausgestaltung der Voruntersuchung kann eine Topographie durchgeführt werden, d.h. eine Vermessung der Augenoberfläche bzw. einer etwaigen Hornhautverkrümmung und Erfassung der individuell unterschiedlichen Höhen und Krümmungswerte. Damit können auch evtl. bestehende Hornhauterkrankungen oder Vernarbungen der Hornhaut festgestellt werden. Bei den voranstehenden Ausgestaltungen der Voruntersuchung handelt es sich um die Grundlagen zur Bestimmung der Ausgestaltung der erfindungsgemäßen Kontaktlinse, die auf den Patienten individuell anzupassen ist.

Anhand der voranstehend beschriebenen Pachymetrie, Biometrie und/oder der Cornea-Topographie, kann die Ziel Topographie der Cornea ermittelt werden, welche zum gewünschten reaktiven Ergebnis nach der Behandlung führen soll. Hierbei wird quasi eine mikrogenaue Vektoren-Landkarte der Cornea erstellt - im "Ist"-Zustand und im idealen "Soll"-Zustand (emmetropen Zustand). Dieser "Soll"-Zustand kann so definiert sein, dass etwaige korrigierbare Astigmatismen und vor allen Dingen die Zielrefraktion (scharfe Abbildung auf der Netzhaut, unter Berücksichtigung Augenlänge etc.) erreicht wird.

Diese Ziel Topographie kann als "Negativ-Abdruck" (analog einer Spritzguss-Form) zur Grundlage der zu fertigenden patientenindividuellen Kontaktlinse, insbesondere der Verformungsseite, genutzt werden. Die Kontaktlinse kann im Spritzgussverfahren bzw. Mikrospritzgussverfahren oder auch durch spanende Verfahren, beispielsweise Drehen oder Fräsen, oder auch mittels 3D-Druckverfahren hergestellt werden.

Die patientenindividuelle Kontaktlinse ist in jedem Fall - aufgrund der inneren Kurvatur bzw. Ausgestaltung der Verformungsseite - in der Lage, mittels Ansaugen der Cornea, die gewünschte Aufsteilung (Hyperopie = weitsichtiges Auge) oder Abflachung (Myopie = kurzsichtiges Auge) zu gewährleisten und stellt somit eine einfache Alternative zu einer LASIK-Behandlung dar. Denkbar ist auch die Korrektur von Astigmatismus und eventuell eines multifokalen CrossLinken, d.h. die Erzeugung von Zonen für Fern- und Nahsicht an der Cornea.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen, ersten Ansicht ein Ausführungsbeispiel eines erfindungsgemäßen ophthalmologischen Operationssets umfassend eine erfindungsgemäße Kontaktlinse,
- Fig. 2: in einer schematischen, zweiten Ansicht das Ausführungsbeispiel gemäß Fig. 1,
- Fig. 3: in einer schematischen Darstellung einen Ausschnitt aus einem menschlichen oder tierischen Auge, auf das ein Vernetzungsmittel eines erfindungsgemäßen ophthalmologischen Operationssets aufgebracht wird,
- Fig. 4: in einer weiteren schematischen Darstellung einen Ausschnitt aus einem menschlichen oder tierischen Auge, auf das eine erfindungsgemäße Kontaktlinse eines erfindungsgemäßen ophthalmologischen Operationssets aufgebracht ist,
- Fig. 5: die Darstellung gemäß Fig. 4, wobei zwischen der Cornea und der Verformungsseite ein Unterdruck vorliegt.

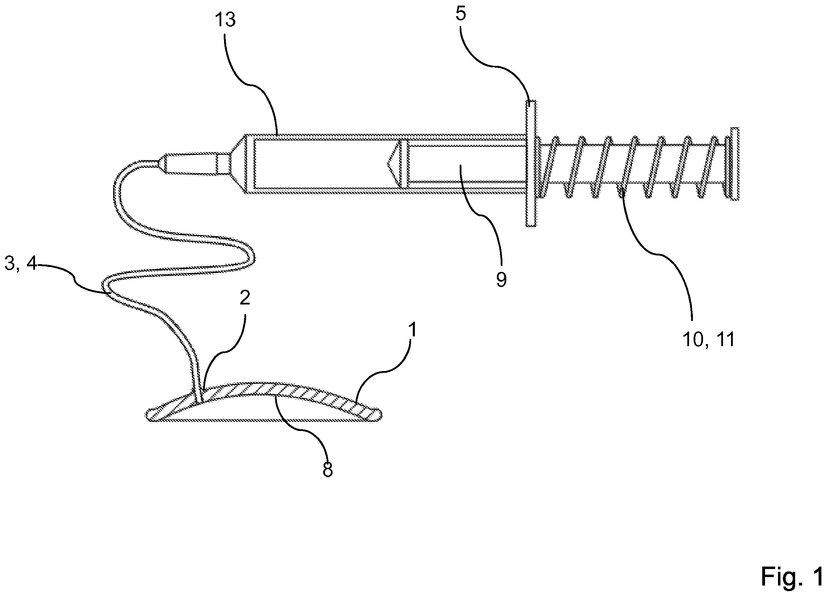

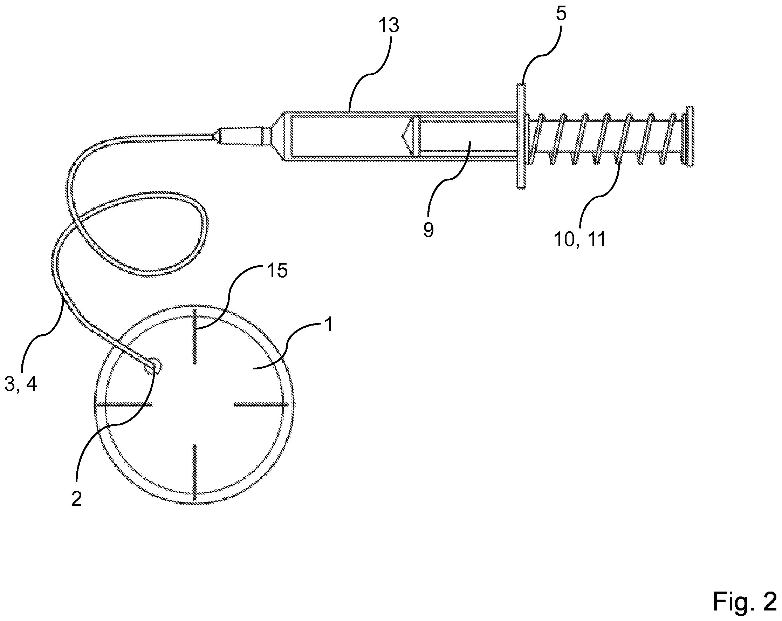

In den Fig. 1 und 2 ist ein Ausführungsbeispiel einer erfindungsgemäßen Kontaktlinse 1 eines erfindungsgemäßen ophthalmologischen Operationssets dargestellt. Die Kontaktlinse 1 ist aus einem formstabilen Material hergestellt und weist als Anschluss 2 ausgebildete Mittel zur Bereitstellung von Unterdruck auf, mit dem ein als Fluidverbindungselement 3 dienender Schlauch 4 gekoppelt ist. Über den Schlauch 4 ist die Kontaktlinse 1 mit einer Spritze 5 verbunden, die zur Erzeugung von Unterdruck zwischen einer in den Fig. 1 und 2 nicht dargestellten Cornea 6 des zu behandelnden Auges 7 und der Verformungsseite 8 der Kontaktlinse 1 dient.

Die Verformungsseite 8 ist individuell an das zu behandelnde Auge angepasst, entspricht nämlich im Wesentlichen dem Negativ einer emmetropen Corneaform.

Der Spritzenkolben 9 ist über ein elastisches Element 10, nämlich eine Feder 11 vorgespannt. Aus Fig. 2 geht hervor, dass die Kontaktlinse 1 eine Markierung 15 in Form eines Fadenkreuzes bzw. absehen aufweist. Dadurch ist die exakte Positionierung der Kontaktlinse 1 auf dem Auge 7 für den Operateur auf einfache Weise möglich.

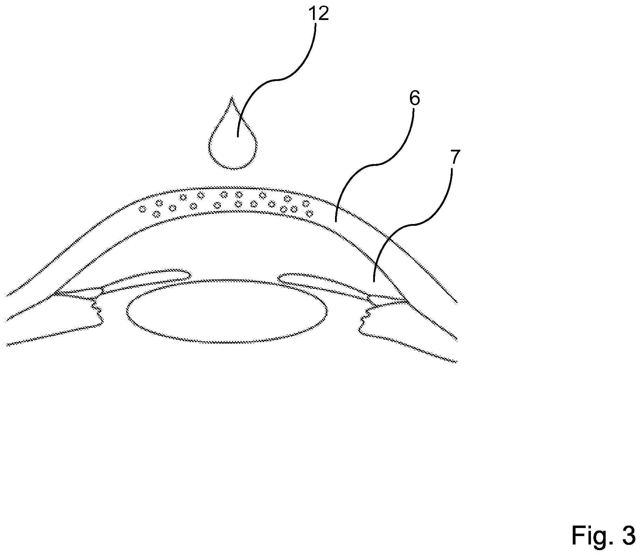

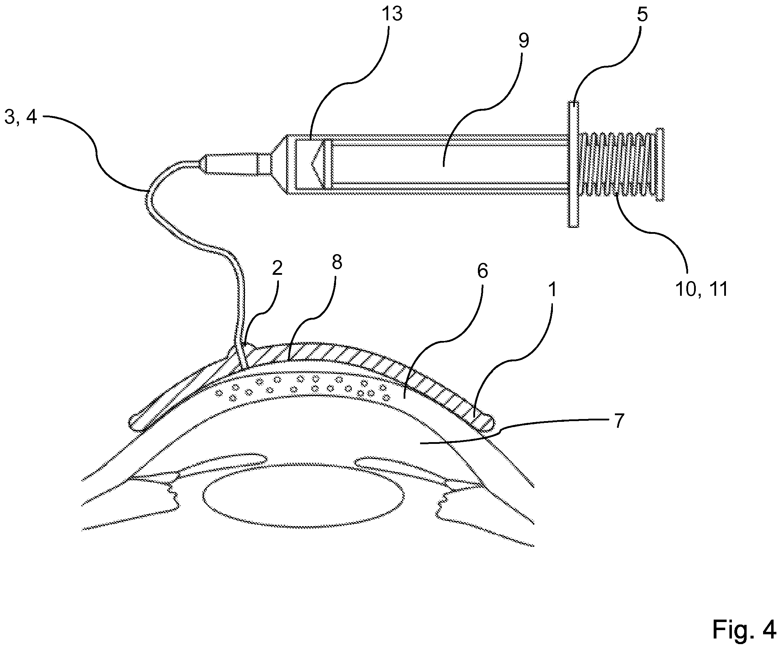

Die Verwendung des erfindungsgemäßen Operationssets wird anhand der Fig. 3 bis 5 erläutert. Zunächst wird das Vernetzungsmittel 12 auf die Cornea 6 aufgebracht, insbesondere aufgetröpfelt. In einem nächsten Schritt wird die an das Auge 7 individuell angepasste Kontaktlinse 1 eingesetzt, wie dies in Fig. 4 dargestellt ist. In Fig. 4 ist deutlich gezeigt, dass der Spritzenkolben 9 gegen die Feder 11 gedrückt ist, so dass zumindest kein wesentlicher Unterdruck zwischen der Cornea 6 und der Verformungsseite 8 herrscht.

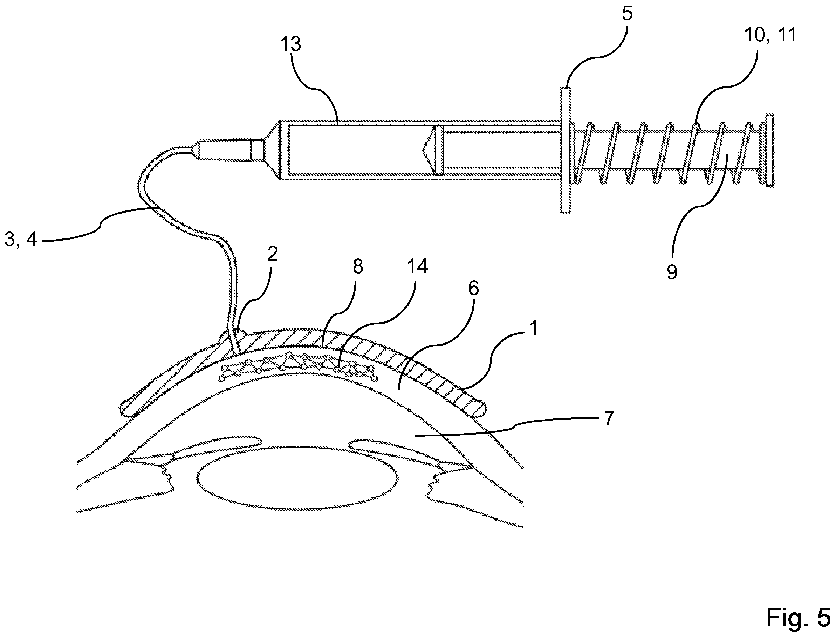

Wenn der Spritzenkolben 9 gelöst bzw. von dem Operateur losgelassen wird, drückt die Feder 11 den Spritzenkolben 9 aus dem Spritzenzylinder 13 heraus, so dass ein definierter Unterdruck zwischen der Verformungsseite 8 und der Cornea 6 herrscht. Dieser Zustand ist in Fig. 5 gezeigt, aus der auch hervorgeht, dass die Cornea 6 durch den Unterdruck an die Verformungsseite 8 angesaugt wird. Somit ist die Cornea 6 in die gewünschte emmetrope Corneaform gebracht und gehalten, in der das Vernetzungsmittel 12 wirken kann. Die Wirkweise des Vernetzungsmittels 12 ist in Fig. 5 durch die Struktur 14 dargestellt, die die Vernetzung der Collagenfasern des Auges verbildlicht.

Nachdem das Vernetzungsmittel 12 gewirkt hat, kann der Unterdruck gelöst und die Kontaktlinse 1 aus dem Auge entfernt werden. Durch das Vernetzungsmittel 12 wird der biomechanische Widerstand der Cornea 6 derart erhöht, dass die emmetrope Corneaform zumindest für einen längeren Zeitraum erhalten bleibt.

Hinsichtlich weiterer vorteilhafter Ausgestaltungen des erfindungsgemäßen Operationssets wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Operationssets lediglich zur Erörterung der beanspruchten Lehre dienen, diese jedoch nicht auf die Ausführungsbeispiele einschränken.

### Bezugszeichenliste

- 1: Kontaktlinse
- 2: Anschluss
- 3: Fluidverbindungselement
- 4: Schlauch
- 5: Spritze
- 6: Cornea
- 7: Auge
- 8: Verformungsseite
- 9: Spritzenkolben
- 10: elastisches Element
- 11: Feder
- 12: Vernetzungsmittel
- 13: Spritzenzylinder
- 14: Struktur
- 15: Markierung

## Patentansprüche

1. Ophthalmologisches Operationsset umfassend mindestens eine Kontaktlinse (1) und mindestens ein Vernetzungsmittel (12) für die Cornea (6), wobei eine der Cornea (6) zugewandte Verformungsseite (8) der Kontaktlinse (1) als Negativ einer zumindest im Wesentlichen emmetropen Corneaform ausgebildet ist und wobei die Kontaktlinse (1) Mittel (6) zur Bereitstellung von Unterdruck zwischen der Kontaktlinse (1) und dem die Kontaktlinse (1) tragenden Auge (7) aufweist, so dass die Cornea an die Verformungsseite (8) ansaugbar und somit in die zumindest im Wesentlichen emmetrope Corneaform bringbar ist,
**dadurch gekennzeichnet, dass**
das Vernetzungsmittel (12) als Teil einer pharmazeutischen Zusammensetzung vorliegt, die für die Verabreichung an bzw. auf das Auge geeignet ist, umfassend:
(i) einen wässrigen Träger; und
(ii) das nicht-toxische, wasserlösliche Vernetzungsmittel, umfassend zwei oder mehrere Carboxylgruppen und/oder eine oder mehrere Anhydridgruppen und/oder eine oder mehrere Estergruppen oder ein pharmazeutisch annehmbares Salz davon, gelöst in dem wässrigen Träger;
wobei die Zusammensetzung einen pH im Bereich von 6 bis 9 aufweist, wobei das nicht-toxische, wasserlösliche Vernetzungsmittel nicht Glutarsäureanhydrid ist.

2. Ophthalmologisches Operationsset nach Anspruch 1, **dadurch gekennzeichnet, dass** die Mittel (6) als Anschluss (6) zur Kopplung mit einem Fluidverbindungselement (3), insbesondere einem Schlauch (4), ausgebildet sind.

3. Ophthalmologisches Operationsset nach Anspruch 2, der Anschluss (2) eine form- und/oder kraftschlüssige Verbindung mit einem Fluidverbindungselement (3), insbesondere einem Schlauch (4), ermöglicht.

4. Ophthalmologisches Operationsset nach Anspruch 2 oder 3, **dadurch gekennzeichnet, dass** der Anschluss (2) als Bajonettanschluss oder als Luer Anschluss ausgebildet ist.

5. Ophthalmologisches Operationsset nach einem der Ansprüche 2 bis 4, **dadurch gekennzeichnet, dass** eine Spritze (5) mit einem Fluidverbindungselement (3), insbesondere einem Schlauch (4), angeordnet ist, das mit dem Anschluss (2) verbindbar ist, um den Unterdruck zwischen der Verformungsseite (8) und der Cornea (6) zu erzeugen.

6. Ophthalmologisches Operationsset nach Anspruch 5, **dadurch gekennzeichnet, dass** die Spritze (5) über ein elastisches Element (10) vorgespannt ist, so dass ein definierter Unterdruck erzeugbar ist.

7. Ophthalmologisches Operationsset nach einem der Ansprüche 1 bis 6, **dadurch gekennzeichnet, dass** an der Kontaktlinse (1) mindestens eine Markierung (15) als Positionierhilfe ausgebildet ist.

8. Ophthalmologisches Operationsset nach einem der Ansprüche 1 bis 7, **dadurch gekennzeichnet, dass** die Verformungsseite (8) derart ausgebildet ist, dass an der Cornea (6) mindestens eine Zone zur Korrektur von Fernsicht und mindestens eine Zone zur Korrektur von Nahsicht erzeugbar sind.

9. Ophthalmologisches Operationsset nach einem der Ansprüche 1 bis 8, **dadurch gekennzeichnet, dass** das Vernetzungsmittel (12) als Vernetzungsflüssigkeit in einer Dosiereinrichtung vorliegt.

10. Ophthalmologisches Operationsset nach Anspruch 9, **dadurch gekennzeichnet, dass** es sich bei der Dosiereinrichtung um eine Spritze oder eine Pipette handelt.

11. Ophthalmologisches Operationsset nach einem der Ansprüche 1 bis 10, **dadurch gekennzeichnet, dass** mindestens ein Lidsperrer angeordnet ist.

12. Ophthalmologisches Operationsset nach einem der Ansprüche 1 bis 11, **dadurch gekennzeichnet, dass** mindestens ein Aufnahmemittel, insbesondere ein Tupfer, angeordnet ist, um überschüssige Flüssigkeiten bzw. überschüssiges Vernetzungsmittel von dem Auge (7) abzutragen.

## Claims

1. Ophthalmological operation set comprising at least one contact lens (1) and at least one cross-linking agent (12) for the cornea (6), wherein a deformation side (8), facing the cornea (6), of the contact lens (1) is in the form of a negative of an least substantially emmetropic cornea form and wherein the contact lens (1) has means (6) for providing reduced pressure between the contact lens (1) and the eye (7) which wears the contact lens (1) so that the cornea can be drawn in at the deformation side (8) and can consequently be brought into the at least substantially emmetropic cornea form,
**characterised in that**
the cross-linking agent (12) is present as part of a pharmaceutical composition which is suitable for administration in or on the eye, comprising:
(i) an aqueous carrier; and
(ii) the non-toxic, water-soluble cross-linking agent comprising two or more carboxyl groups and/or one or more anhydride groups and/or one or more ester groups or a pharmaceutically acceptable salt thereof in a state dissolved in the aqueous carrier;
wherein the composition has a pH in the range from 6 to 9,
wherein the non-toxic, water-soluble cross-linking agent is not glutaric acid anhydride.

2. Ophthalmological operation set according to claim 1, **characterised in that** the means (6) are in the form of a connection (6) for coupling to a fluid connection element (3), in particular a hose (4).

3. Ophthalmological operation set according to claim 2, the connection (2) enables a positive-locking and/or non-positive-locking connection to a fluid connection element (3), in particular a hose (4).

4. Ophthalmological operation set according to claim 2 or 3, **characterised in that** the connection (2) is in the form of a bayonet connection or a Luer connection.

5. Ophthalmological operation set according to any one of claims 2 to 4, **characterised in that** there is arranged a syringe (5) having a fluid connection element (3), in particular a hose (4), which can be connected to the connection (2) in order to produce the reduced pressure between the deformation side (8) and the cornea (6).

6. Ophthalmological operation set according to claim 5, **characterised in that** the syringe (5) is pretensioned by means of a resilient element (10) so that a defined reduced pressure can be produced.

7. Ophthalmological operation set according to any one of claims 1 to 6, **characterised in that** at least one marking (15) acting as an auxiliary positioning member is formed on the contact lens (1).

8. Ophthalmological operation set according to any one of claims 1 to 7, **characterised in that** the deformation side (8) is constructed in such a manner that at least one zone for correction of distance vision and at least one zone for correction of near vision can be produced on the cornea (6).

9. Ophthalmological operation set according to any one of claims 1 to 8, **characterised in that** the cross-linking agent (12) is present as a cross-linking fluid in a metering device.

10. Ophthalmological operation set according to claim 9, **characterised in that** the metering device is a syringe or a pipette.

11. Ophthalmological operation set according to any one of claims 1 to 10, **characterised in that** at least one lid speculum is arranged.

12. Ophthalmological operation set according to any one of claims 1 to 11, **characterised in that** at least one absorbent means, in particular a swab, is arranged in order to remove excess fluids or excess cross-linking agent from the eye (7).

## Revendications

1. Kit d'opération ophtalmologique comprenant au moins une lentille de contact (1) et au moins un agent de réticulation (12) pour la cornée (6), dans lequel un côté de déformation (8) de la lentille de contact (1) tourné vers la cornée (6) est conçu comme le négatif d'une forme de cornée au moins sensiblement emmétrope et la lentille de contact (1) présentant des moyens (6) pour générer une dépression entre la lentille de contact (1) et l'œil (7) portant la lentille de contact (1), de sorte que la cornée peut être aspirée contre le côté de déformation (8) et ainsi être amenée à la forme de cornée au moins sensiblement emmétrope,
**caractérisé en ce que**
l'agent de réticulation (12) fait partie d'une composition pharmaceutique appropriée pour l'administration dans ou sur l'œil, comprenant :
(i) un support aqueux ; et
(ii) l'agent de réticulation non toxique et soluble dans l'eau,
comprenant deux ou plusieurs groupes carboxyle et/ou un ou plusieurs groupes anhydride et/ou un ou plusieurs groupes ester ou un sel pharmaceutiquement acceptable de ceux-ci, dissous dans le support aqueux ;
dans lequel la composition ayant un pH dans la plage de 6 à 9, dans laquelle l'agent de réticulation anhydre non toxique n'est pas l'anhydride glutarique.

2. Kit chirurgical ophtalmologique selon la revendication 1, **caractérisé en ce que** les moyens (6) sont réalisés sous la forme d'un raccord (6) pour le couplage avec un élément de liaison fluidique (3), plus particulièrement un tuyau (4).

3. Kit chirurgical ophtalmologique selon la revendication 2, le raccord (2) permet une liaison par complémentarité de forme et/ou de force avec un élément de liaison fluidique (3), plus particulièrement un tuyau (4).

4. Kit chirurgical ophtalmologique selon la revendication 2 ou 3, **caractérisé en ce que** le raccord (2) est conçu comme un raccord à baïonnette ou un raccord Luer.

5. Kit chirurgical ophtalmologique selon l'une des revendications 2 à 4, **caractérisé en ce qu'**une seringue (5) est munie d'un élément de liaison fluidique (3), plus particulièrement un tuyau (4), qui peut être relié au raccord (2) pour créer la dépression entre le côté de la déformation (8) et la cornée (6).

6. Kit chirurgical ophtalmologique selon la revendication 5, **caractérisé en ce que** la seringue (5) est précontrainte par un élément élastique (10), de sorte qu'une dépression définie peut être générée.

7. Kit chirurgical ophtalmologique selon l'une des revendications 1 à 6, **caractérisé en ce qu'**au moins un repère de positionnement (15) est prévu sur la lentille de contact (1).

8. Kit de chirurgie ophtalmique selon l'une des revendications 1 à 7, **caractérisé en ce que** la face déformable (8) est conçue de telle sorte qu'au moins une zone de correction de la vision de loin et au moins une zone de correction de la vision de près peuvent être créées sur la cornée (6).

9. Kit chirurgical ophtalmologique selon l'une des revendications 1 à 8, **caractérisé en ce que** l'agent de réticulation (12) se présente sous forme de liquide de réticulation dans un dispositif de dosage.

10. Kit chirurgical ophtalmologique selon la revendication 9, **caractérisé en ce que** le dispositif de dosage est une seringue ou une pipette.

11. kit chirurgical ophtalmologique selon l'une des revendications 1 à 10, **caractérisé en ce qu'**au moins un dispositif de blocage des paupières est prévu.

12. Kit chirurgical ophtalmologique selon l'une des revendications 1 à 11, **caractérisé en ce qu'**au moins un moyen de collecte, plus particulièrement un écouvillon, est prévu pour évacuer de l'œil (7) les liquides ou l'agent de réticulation en excès.
